# EUROPEAN PATENT SPECIFICATION

(11) **EP 2 258 744 B1**
(45) Date of publication and mention of the grant of the patent: **31.07.2013**
(21) Application number: 10179153.1
(22) Date of filing: 18.05.2004
(51) Int. Cl.: C09D 175/16, C08G 63/91, C08G 63/82, C08G 63/87, C08G 18/68, C08G 18/42, C08G 18/36, C08G 18/24, C12P 7/62

(54) **Enzyme Catalyzed Polyesters and Polyol Polymers**
Enzym-katalysierte Polyester- und Polyolpolymere
Polymères de polyol et de polyester catalysés par enzyme

(30) Priority: 30.05.2003 US 448794
(43) Date of publication of application: 08.12.2010
(62) Divisional of application: 04011762.4
(73) Proprietor: Armstrong World Industries, Inc., Lancaster Pennsylvania 17603 (US)
(72) Inventor: Tian, Dong, Lancaster, PA 17601 (US); Ross, Jeffrey S., Lancaster, PA 17603 (US); Price, Courtney R., Dillsburg, PA 17019 (US)
(74) Representative: von Füner, Nicolai

(56) References cited:
- EP-A- 0 610 718
- WO-A-99/46397
- US-A- 5 631 343
- US-A1- 2004 019 178

## Description

This invention is generally in the area of the preparation of polyester polyols as well as enzyme catalyzation of acrylated polyester polyols, and coating compositions including polyester polyols and/or acrylated polyester polyols prepared by enzyme catalyst, providing stable coatings that offer several advantages relative to the use of heavy metal or strong acid catalysts to prepare the polyester polyols.
A limitation of using acidic and/or heavy metal catalysts is that the residues of these catalysts can accelerate the decomposition of the resulting polymers.

WO 99/46397 relates to the enzymatic synthesis of a linear polyester made of polybasic carboxylic acids and polyhydric alcohols in the presence of an effective quantity of a lipase.

US 5 631 343 relates to a process for producing a polyester consisting of residues of an aliphatic dicarboxylic acid and an aliphatic diol in the absence of a solvent and in the presence of a lipase.

It would be advantageous to provide methods for generating polyester polyols, including acrylated polyester polyols, using enzymatic catalysts, and providing coating compositions including these acrylated polyester polyols or components derived from these acrylated polyester polyols that are essentially free from strong acid or heavy metal residues. The present invention provides such methods and compositions.

Methods for synthesizing saturated and unsaturated polyester polyols are disclosed. The methods are environmentally-friendly and provide environmentally-friendly compositions that do not contain heavy metals or strong acid catalyst residues such as are present when heavy metals and/or strong acids are used as catalysts to manufacture these materials.

In the present invention, the polyester polyols are prepared by the esterification of a di- or polycarboxylic acid with a polyhydric alcohol or a polyhydric alcohol modified to include one or more acrylate moieties, using an enzymatic catalyst, advantageously avoiding the use of an acidic or heavy metal catalyst. The resulting polyester polyol can be acrylated if desired.

Polyol polymer and acrylated polyol polymer-containing compositions that are essentially free of acidic and heavy metal catalyst residues, and which can contain a lipase and/or lipase residues, are also disclosed.

Urethane acrylates prepared using these polyol polymers and/or acrylated polyol polymers and methods of preparing the urethane acrylates are also disclosed. The urethane acrylates are prepared by reacting one or more hydroxy moieties on the polyol polymers with one or more isocyanate moieties on a di- and/or polyisocyanate to form urethane linkages. In one embodiment, the polyol polymer includes at least one (meth)acrylate moiety. In another embodiment, at least one isocyanate moiety in the polyisocyanate is reacted with a hydroxy moiety on a compound that includes at least one hydroxy moiety and at least one (meth)acrylate moiety. In either embodiment, the resulting urethane includes at least one (meth)acrylate moiety. When the polyol polymer is prepared using a lipase catalyst rather than an acidic or heavy metal catalyst, the urethane acrylate is essentially free of acidic or heavy metal catalyst residues.

Coating compositions including the polyol polymers and acrylated polyol polymers and/or urethanes and urethane acrylates prepared using these polyol polymers or acrylated polyol polymers are also disclosed. The coating compositions can be 100% solids coating compositions, water-based coating compositions or solvent-based coating compositions. The compositions can include flatting agents, reactive diluents, photoinitiators, and/or other components suitable for use in coating compositions. In one embodiment, the coatings prepared using the coating compositions are essentially free of acidic and/or heavy metal catalyst residues. The coating compositions can be used to coat surface coverings, such as floor, wall, and ceiling coverings, and such coated floor, wall and ceiling coverings are also disclosed. The "catalyst free" coating compositions can also be water-based, solvent-based and 100% solids UV-curable coating compositions, and can be used to prepare high-performance coatings. The coatings prepared from these coating compositions have improved stability relative to coatings prepared using polyol polymers, acrylated polyol polymers, urethanes or urethane acrylates prepared using heavy metal and/or acid catalysts and that include residues of these catalysts.

In addition to coatings, the polyol polymers and acrylated polyol polymers and/or urethanes and urethane acrylates prepared using these polyol polymers or acrylated polyol polymers, can be used as one or more components in the preparation of various articles of manufacture, such as food wraps, children's toys. Additionally, these polyol polymers can be used to prepare a variety of urethane products that find application in foams, biomedical materials, thermoplastic polyurethanes.

The polyol polymers and acrylated polyol polymers, methods for preparing these compounds, urethanes and urethane acrylates prepared using these compounds, coating compositions including these compounds and/or urethanes and urethane acrylates prepared using these compounds, and surface coverings and other articles of manufacture prepared from these compounds and compositions, are discussed in more detail below.

The polyol compositions described herein are essentially free of residual acid and/or heavy metals (i.e., the chemistry is "green chemistry" and is environmental-friendly), relative to those prepared using heavy metal and/or acid catalysts. Further, coatings that include these compounds have relatively improved long-term stability, including hydrolysis stability, color stability and light and heat stability when compared to polyol polymers prepared using a catalyst selected from the group consisting of a strong acid catalyst and a metallic catalyst.

Using enzymatic catalysts, the method can be performed at lower temperatures than when heavy metals and/or strong acid catalysts are used. For example, the processing temperature using the enzymatic catalysts is in the range of 70-90°C, whereas a temperature range of 120-160°C is used in connection with acid and/or heavy metal catalysts, with similar reaction times. This results in significant energy savings, and can also result in less color in the final products.

The resulting method provides saturated and unsaturated macro monomers. The saturated and unsaturated macro monomers have a wide application in the coating market. Also, polylactone polyols are one of the major components in the polyurethane market (including thermoplastic polyurethane). For example, polylactone polyols can be reacted with isocyanates and hydroxy-acrylates to form the urethane acrylates in several commercial coating formulations, including Armstrong World Industries' radiation curable Duracote™ coating formulations. In particular, the polyols can be used with a hydroxyacrylate such as Union Carbide's Tone M100 and an isocyanate such as BASF's Desmodur® 3300.

Finally, the ability to avoid metal or acid catalyst residues in UV-curable compositions provides certain additional advantages, such as relatively improved heat and light stability when compared to similar compositions including such residues. Although catalyst residue is not known to pose significant health or safety risks with finished floor coverings or polyurethane coatings, the avoidance of such residues is advantageous when the materials are used in food packaging, children's toys, and medical and biomedical implants. The compositions described herein are essentially free of acidic or heavy metal catalyst residues (i.e., include less than about 50 ppm of these residues).

### I. Production of Polyol Polymers

In the present invention, di- and/or polycarboxylic acids are reacted with compounds including more than one hydroxy moiety to form polyester polyols. The esterification reaction is catalyzed by a lipase.

The polyester polyols include one or more (meth)acrylate moieties, which are provided by end-capping the polyester polyols with (meth)acrylate moieties, ideally using a lipase enzyme as the catalyst, and by providing a (meth)acrylate moiety in the hydroxy moiety-containing compounds used in the ring-opening polymerization or the esterification reaction.

The individual reaction components are described in detail below.

### A. Di-and Polycarboxylic Acids

Di- and polycarboxylic acids (also referred to herein as dibasic and polybasic carboxylic acids) can also be formed into polyester polyols by reaction with compounds including two or more hydroxy moieties (i.e., di- and/or polyols). Examples of dibasic and polybasic carboxylic acids that can be used to form the polyester polyols include phthalic acid, phthalic anhydride, isophthalic and terephthalic acid, maleic acid, maleic anhydride, succinic acid, succinic anhydride, adipic, trimellitic acid, trimellitic anhydride, pimelic, suberic, azelaic and sebacic acid, fumaric and citraconic acid, glutaric acid, glutaric anhydride, pyromellitic acid and pyromellitic anhydride, tetrahydrophthalic acid, tetrahydrophthalic anhydride.

### B Enzymatic Catalysts

Examples of suitable enzymatic lipase catalysts (lipases) include porcine pancreatic lipase (PPL), *Candida cylindracea* lipase (CCL), *Pseudomonas fluorescence* lipase (PFL), *Rhizopus javanicuc* lipase (RJL), *Rhizopus delemar* lipase (RDL) and Novozyme 435^{™}.

### C Solvent Systems

The reaction can be performed in any suitable solvent system. The solvent ideally does not include a significant amount of water beyond that required for the enzyme to function, since the water can compete with the polyol initiators to initiate polymerization of the lactones. The solvent ideally does not include a significant amount of alcohols other than the polyol initiators for the same reason. In those embodiments where the final product is a liquid at the reaction temperature, the reaction can be run neat (i.e., without added solvent).

Ionic liquids and/or supercritical fluid carbon dioxide have both been used as solvents for enzymatic reactions, and can be used in the methods described herein. Organic solvents, such as hexane, heptane, toluene, xylene, can also be used.

### II. Acrylation of Polyols.

. A second way to incorporate acrylate moieties using the lipase catalysts is to first obtain polyester polyols, and then acrylate one or more hydroxyl moieties on the polyester polyols using (meth)acrylic acid using the lipase catalyst. Advantageously, the polyester polyols that is used is also prepared using a lipase, so that it does not include any acidic or heavy metal catalyst residues.

### Polyol Polymers

The polyester polyols described herein include at least one or two free hydroxyl moieties per molecule, and include polyesters with or without ether moieties, and polyethers with or without ester moieties. The hydroxyl-containing polyesters can be formed by esterifying dibasic or polybasic carboxylic acids with dihydric or polyhydric alcohols. In general, the carboxylic acid component used to esterify the hydroxyl-containing polyesters can be dibasic, tribasic and/or tetrabasic, aliphatic and/or aromatic C₃-₃₆ carboxylic acids, and their esters and anhydrides. The polyester polyols can be identical to those prepared by ring-opening polymerization of lactones, provided they include at least one or two free hydroxyl moieties per molecule.

Examples of dihydric and polyhydric alcohols suitable as starting materials for preparing polyesters include dihydric to hexahydric alcohols. Representative diols include, , ethylene glycol, propylene glycol, 1,4-butanediol, 2-ethyl-1,4-butanediol, 1,5-pentanediol, 2-methyl-1,5-pentanediol, 1,6-hexanediol, dimethylolcyclohexane, neopentyl glycol, triols such as trimethylolethane, trimethylolbutane, trimethylolpropane, glycol, tetraols such as pentaerythritol and ditrimethylolpropane, hexols, such as erythritol and sorbitol. Other polyesterols that can be used include polylactonediols, - triols and -tetraols, as mentioned above. The polyols also can be the alkoxylates of the above-mentioned dihydric and/or polyhydric alcohols, including ethoxylated, propoxylated and mixed ethoxylated and propoxylated dihydric to hexahydric alcohols and polyesterols. The degree of alkoxylation is generally from 1 to 300, preferably from 2 to 150. Further, the polyols can be polyalkylene glycols and the polyaddition polymers of cyclic ethers, such as polytetrahydrofuran. Examples of polyalkylene glycols include polyethylene glycol, polypropylene glycol and polyepichlorohydrins.

Other polyester polyols that can be used include copolymers comprising in copolymerized form at least one of the above mentioned monomeric, oligomeric and/or polymeric components. Polyesters of the above-mentioned dibasic and/or polybasic carboxylic acids and alcohols with terminal carboxyls or hydroxyls, and polyetherols, such as the above mentioned alkoxylates, polyalkylene glycols and polymers of cyclic ethers, also can be used. Furthermore, the polyols can be mono- or multi-functional alcohols, such as diols, triols, tetraols, hexols. Representative examples include tridecyl alcohol, iso-octanol, ethylene glycol, propylene glycol, 1,4-butanediol, 2-ethyl-1,4-butanediol, 1,5-pentanediol, 2-methyl-1,5-pentanediol, 1,6-hexanediol, dimethylolcyclohexane, neopentyl glycol, trimethylolethane, trimethylolbutane, trimethylolpropane, glycol, pentaerythritol, ditrimethylolpropane, erythritol and sorbitol.

### III. Urethanes and Urethane Acrylates Prepared Using Saturated and Unsaturated Polyester Polyols

The saturated and unsaturated polyester polyols described herein can be used to prepare urethanes and urethane acrylates. Where the polyester polyols are saturated, the reaction of a di- or polyisocyanate with the hydroxyl moiety in the polyol polymer forms a urethane. In this reaction, a chain extender, i.e. an amine, may be used. Where the polyester polyols include one or more (meth)acrylate moieties, the reaction of a isocyanate in a di- or polyisocyanate with a hydroxy moiety in the polyester polyols forms a urethane linkage, and the urethanes already include a (meth)acrylate functionality. Where the polyester polyols do not include one or more (meth)acrylate moieties, one or more of the isocyanate moieties can be reacted with a compound that includes a hydroxy moiety and a (meth)acrylate moiety, such as a hydroxy acrylate, so that the urethane molecule includes one or more (meth)acrylate moieties.

The isocyanate compounds used for preparing the urethane acrylates can be selected from the compounds having a linear saturated hydrocarbon, cyclic saturated hydrocarbon, or aromatic hydrocarbon structure. Such isocyanate compounds can be used either individually or in combinations of two or more. The number of isocyanate moieties in a molecule is usually from 1 to 6, and preferably from 1 to 3.

Polyurethanes produced from aromatic isocyanates tend to turn yellowish over time, albeit without a significant loss in mechanical properties. For this reason, in coating applications, it may be desirable to use aliphatic isocyanates. The most widely used aliphatic diisocyanates are 1,6-hexane diisocyanate (HDI), isophorone diisocyanate (IPDI), dicyclohexane diisocyanate (HMDI) also known as hydrogenated MDI, and meta-tetramethylxylene diisocyanate (TMXDI). Additional nonaromatic isocyanates include 1,6,11- undecane triisocyanate, lysine-ethylester triisocyanate, transcyclohexane diisocyanate, and tetramethylxylylene diisocyanate. Toluene diisocyanate and xylylene diisocyanate are examples of aromatic isocyanates.

Representative examples of commercially available polyisocyanate compounds include A-1310 and Y-5187 manufactured by Nippon Unicar Co., Ltd.; Calenz MOI manufactured by Showa Denko Co., Ltd.; TDI-80/20, TDI-100, MDI-CR100, MDI-CR300, MDI-PH, and NDI manufactured by Mitsui-Xisso Urethane Co., Ltd.; Coronate T, Millionate MT, Millionate MR, and HDI manufactured by Nippon Polyurethane Industry Co., Ltd.; and Takenate 600 manufactured by Takeda Chemical Industries Co., Ltd.

### IV. Coating Compositions Including the Polyol Polymers and/or Urethane Acrylates Prepared from the Polyol Polymers

The polyester polyols, acrylated polyester polyols and/or urethanes and urethane acrylates prepared from the polyester polyols and acrylated polyester polyols described herein can be used in coating compositions. The coating compositions can also include reactive diluents, photoinitiators, flatting agents, and other components commonly used in coating compositions. The coating compositions can be water-based, organic solvent-based, or 100% solids coating compositions.

### A. Reactive Diluents

The polyester polyols, particularly acrylate polyester polyols, and urethanes and urethane acrylates prepared from the polyester polyols and acrylated polyester polyols, can be combined with suitable reactive diluents to form UV-curable 100 percent solids coating compositions. The reactive diluent(s) are typically low molecular weight (i.e., less than 1000 g/mol), liquid (meth)acrylate-functional compounds. Examples include: tridecyl acrylate, 1,6-hexanediol diacrylate, 1,4-butanediol diacrylate, ethylene glycol diacrylate, diethylene glycol diacrylate, tetraethylene glycol diacrylate, tripropylene glycol diacrylate and ethoxylated derivatives thereof, neopentyl glycol diacrylate, 1,4-butanediol dimethacrylate, poly(butanediol) diacrylate, tetrathylene glycol dimethacrylate, 1,3-butylene glycol diacrylate, tetraethylene glycol diacrylate, triisopropylene glycol diacrylate, triisopropylene glycol diacrylate, and ethoxylated bisphenol-A diacrylate. Another example of a reactive diluent is N-vinyl caprolactam (International Specialty Products). Further examples are the commercially available products from Sartomer, SR 489, a tridecyl acrylate and SR 506, an isobornyl acrylate.

### B. Flatting Agents

Flatting agents are well known to those of skill in the art, and are used to minimize the gloss levels of coatings. Representative flatting agents include silica, nylon, and alumina flatting agents.

### C. Photoinitiators

The compositions can also include a sufficient amount of a free-radical photoinitiator such that the compositions can be UV-cured. Typically, the concentration of photoinitiator is between 1 and 10% by weight, although weight ranges outside of this range can be used. Alternatively, the compositions can be cured using electron beam (EB) curing.

Any compounds that decompose upon exposure to radioactive rays and initiate the polymerization can be used as the photoinitiator in UV-curable compositions including the polyols, acrylated polyols and/or urethane acrylates prepared from the polyols or acrylated polyols. Photosensitizers can be added as desired. The words "radiation" as used in the present invention include infrared rays, visible rays, ultraviolet rays, deep ultraviolet rays, X-rays, electron beams, alpha-rays, beta-rays, gamma-rays. Representative examples of the photoinitiators include, acetophenone, acetophenone benzyl ketal, anthraquinone, 1-hydroxycyclohexylphenyl ketone, 2,2-dimethoxy-2-phenylacetophenone, xanthone compounds, triphenylamine, carbazole, 3-methylacetophenone, 4-chlorobenzophenone, 4,4'-dimethoxybenzophenone, 4,4'-diaminobenzophenone, 2-hydroxy-2-methyl-1-phenylpropan-1-one, 1-(4-isopropylphenyl)-2-hydroxy-2-methylpropan-1-one, xanthone, 1,1-dimethoxydeoxybenzoin, 3,3'-dimethyl-4-methoxybenzophenone, thioxanethone compounds, diethylthioxanthone, 2-isopropylthioxanthone, 2-chlorothioxanthone, 1-(4-dodecylphenyl)-2-hydroxy-2-methylpropan-1-one, 2-methyl-1-[4-(methylthio)phenyl]-2-morpholino-propan-1-one, triphenylamine, 2,4,6-trimethylbenzoyldiphenylphosphineoxide, bis- (2,6-dimethoxybenzoyl)-2,4,4-trimethylpentylphosphine oxide, bisacylphosphineoxide, benzyl dimethyl ketal, fluorenone, fluorene, benzaldehyde, benzoin ethyl ether, benzoin propyl ether, benzophenone, Michler's ketone, 2-benzyl-2-dimethylamino-1- (4-morpholinophenyl)-butan-1-one, 3-methylacetophenone, and 3,3',4,4'-tetra(t-butylperoxycarbonyl)benzophenone (BTTB).

Commercially available photoinitiators include Irgacure® 184, 651, 500, 907, 369, 784 and 2959 (manufactured by Ciba Specialty Chemicals Co., Ltd.), Lucirine TPO (manufactured by BASF), Darocur® 1116 and 1173 (manufactured by Ciba Specialty Chemicals Co., Ltd.), Lucirine TPO (manufactured by BASF), Ubecryl® P36 (manufactured by UCB), and Escacure® KIP150,KIP100F (manufactured by Lamberti).

Representative examples of photosensitizers include triethylamine, diethylamine, N-methyldiethanolamine, ethanolamine, 4-dimethylaminobenzoic acid, methyl 4-dimethylaminobenzoate, ethyl 4-dimethylaminobenzoate, and isoamyl 4-dimethylaminobenzoate, as well as commercially available products such as Ubecryl® P102, 103, 104, 105 (manufactured by UCB).

The photoinitiators are typically present in the range of from 0.01 to 10 wt %, although amounts outside this range can be used. Thermal initiators, such as AIBN and di-t-butyl peroxide can be used in place of or in addition to the photoinitiators.

The coating compositions can be used to coat surface coverings, such as floor, wall and ceiling coverings. The coating compositions can be applied to surface coverings using any application method suitable for use with urethane acrylate coating compositions. Such methods include roll coating, spray coating, dip coating. Such coatings are essentially free of acidic and/or heavy metal catalyst residues, and as such, are more stable than coatings including such residues.

The resulting coated products can be tested for improved stability, including stability to both heat and light. Standard ASTM method F 1514-98 can be used, advantageously with minor modifications. The standard ASTM method calls for evaluating the material at 158°F for 7 days and comparing color differences. Advantageously, this test is extended to 6 weeks, with results being determined at every week interval.

Light stability testing can be evaluated using ASTM F1515, advantageously with minor modifications. The standard ASTM method involves exposing the material to a Xenon light source and reporting any differences in color readings at 100, 200, 300 and 400 hours. Advantageously, this test is extended to 500 and 600 hrs.

Improvements in stability can be evaluated by making identical compositions wherein the only difference in the composition is the catalyst used to make the polyol polymer or acrylated polyol polymer materials. That is, one sample can be prepared via enzymatic catalysis, and the second sample prepared via conventional acid or metal-based catalysis.

### V. Articles of Manufacture Prepared Using the Polyols and Acrylated Polyols and/or Urethanes and Urethane Acrylates Prepared from the Polyols

The polyester polyols, acrylated polyester polyols, urethanes, and/or urethane acrylates can be used to prepare any article of manufacture commonly made using these components. They can be present in plastics used in food packaging, such as food containers and films used to wrap food products, children's toys, medical and biomedical implants, and the like. In one embodiment, the compounds and/or compositions including the compounds are added to a mold and polymerized to form a desired article of manufacture.

The present invention will be better understood with reference to the following nonlimiting examples.

### Comparative Example 1: Preparation of a hydroxyl-terminated polyester triol:

One hundred forty-eight grams of epsilon-caprolactone, 26 grams of trimethylolpropane and 3 grams porcine pancrease lipase (type II) were charged into 250 ml glass reaction vessel fitted with a mechanical stirrer, condenser, thermometer and dry air inlet and outlet tubes. The mixture was stirred and heated to 70°C with blanketed dry air at 0.10 SCFH (0.283 cubic meter/hour at 273.15 °K (0°C) and one atmosphere of pressure (760 torr or 101.325 kilopascals)). The reaction mixture was stirred at 70°C. Gas chromatography analysis was used to monitor epsilon-caprolactone conversion. After 4 hours of reaction time, the epsilon-caprolactone conversion was 98.5% complete. After 6 hours, the reaction was 98.7% complete. The reaction mixture was then cooled down to room temperature. The final product was collected by filtering off the enzyme catalyst.

### Comparative Example 2: Preparation of Unsaturated polyols

Six hundred twenty-six grams of epsilon-caprolactone, 318 grams of 2-hydroxyethyl acrylate, 0.68 grams monomethyl ether hydroquinone (p-methoxyphenol) and 9 grams Novozyme-435 were charged into 1L glass reaction vessel fitted with a mechanical stirrer, condenser, thermometer and dry air inlet and outlet tubes. The mixture was stirred and heated to 70°C with blanketed dry air (0.10 SCFH (0.283 cubic meter/hour at 273.15 °K (0°C) and one atmosphere of pressure (760 torr or 101.325 kilopascals))). The reaction mixture was stirred at 70°C. Gas chromatography analysis was used to monitor epsilon-caprolactone conversion. After 4 hours, the epsilon-caprolactone conversion was 99.6% complete. After 6 hours, there was no change in the amount of conversion. The reaction mixture was then cooled down to room temperature. The final product was collected by filtering off the enzyme catalyst.

### Comparative Example 3: Preparation of Multi-functional unsaturated polyols

One hundred twenty four grams of epsilon-caprolactone, 62 grams trimethylolpropane diallyl ether, 0.14 grams monomethyl ether hydroquinone (p-methoxyphenol) and 2 grams Novozyme-435 were charged into 250 ml glass reaction vessel fitted with a mechanical stirrer, condenser, thermometer and dry air inlet and outlet tubes. The mixture was stirred and heated to 70°C with blanketed dry air (0.10 SCFH (0.283 cubic meter/hour at 273.15 °K (0°C) and one atmosphere of pressure (760 torr or 101.325 kilopascals))). The reaction mixture was stirred at 70°C. Gas chromatography analysis was used to monitor epsilon-caprolactone conversion. After 4 hours, the epsilon-caprolactone conversion was 98% complete. There was no change after 6 hours. The reaction mixture was then cooled down to room temperature. The final product was collected by filtering off the enzyme catalyst.

### Example 4: Preparation of acrylated polyester polyol

Two hundred sixty-seven grams of a hydroxy-terminated polyester (a polymer prepared from composition containing 59.3% by weight of 1,6 hexanediol, 15.6% phthalic anhydride, and 25.1 % trimellitic anhydride, where the acid number is less than 5), 92 grams of acrylic acid, 0.04 grams monomethyl ether hydroquinone (p-methoxyphenol), 0.04 grams hydroquinone, 36 grams Novozyme-435 and 65 ml heptane were charged into 1000 ml glass reaction vessel fitted with a mechanical stirrer, D-S trap and water condenser connected to a vacuum system, thermometer and dry air inlet. The mixture was stirred and heated to 90°C with blanketed dry air at 0.10 SCFH (0.283 cubic meter/hour at 273.15 °K (0°C) and one atmosphere of pressure (760 torr or 101.325 kilopascals)). Then, the dry airflow was stopped and vacuum was gradually applied to initiate boiling. The boiling was adjusted to maximum and the aqueous layer formed in phase separation was removed. Additional heptane was added as needed due to heptane loss. After 6 hours, the final product was recovered by vacuum stripping off the solvent and excess acrylic acid, and filtering off the enzyme catalyst. The product, acrylated polyester polyol, had a hydroxy number of 88.

### Example 5: Coating formulation

Polycaprolactone triols, unsaturated polyol and acrylated polyester polyol obtained from Examples 1, 2 & 4 were formulated into a dual (thermal/UV) cure coating. See formulation in Table 1. The coating was coated on a vinyl floor, thermally cured at 190°C for 2 minutes, then UV-cured at 2J/cm². The final coating had very good stain resistance and abrasion resistance without containing any metal residue.

**Table 1**

| | |
|---|---|
| Acrylated polyester polyol (Example 4) | 70.00 gr |
| Unsaturated polyol (Comparative Example 2) | 15.00 gr |
| Polyester polyol (Comparative Example 1) | 15.00 gr |
| 2-hydroxyethyl acrylate | 15.00 gr |
| Resimene® CE-7103 | 51.87 gr |
| DC-57 | 0.50 gr |
| Benzophenone | 3.01 gr |
| KIP-100F | 2.01 gr |
| Nacure® XP-357 | 6.69 gr |

## Claims

1. A coating composition comprising a (meth)acrylate polyester polyol, wherein the polyester polyol is prepared by a method comprising a step selected from the group consisting of:
(a) forming a polyester polyol by esterification of polyfunctional carboxylic acids and polyfunctional hydroxy-containing materials with a (meth)acrylate moiety, and wherein the esterification is catalyzed by enzymatic catalysis,
(b) capping at least one of the end of a polyester polyol with a (meth)acrylate moiety, wherein the end capping reaction is catalyzed by an enzymatic catalyst, and
(c) the combination of steps (a) and (b).

2. The coating composition according to claim 1, wherein the composition is essentially free of acidic catalyst residues and metallic catalyst residues.

3. The coating composition according to claim 1 or 2, wherein the enzyme catalyst comprises a lipase enzyme.

4. A coating composition comprising a compound selected from the group consisting of a urethane monomer, wherein the urethane moiety comprises urethane linkages formed from at least one hydroxy moiety on a polyester polyol according to any of the claims 1 to 3, wherein the polyester polyol being prepared via enzyme-catalyzed esterification, with at least one isocyanate moiety on a polyisocyanate.

5. The coating composition according to claim 4, wherein the composition further comprises a reactive diluent.

6. The coating composition according to claims 4 or 5, wherein the composition further comprises a flatting agent.

7. The coating composition according to any of the claims 4 to 6, wherein the composition further comprises a photoinitiator.

8. A method for preparing the coating composition according to any of claims 1 to 3, comprising
(a) forming a polyester polyol by the esterification of an acid selected from the group consisting of dibasic carboxylic acid, polybasic carboxylic acid, and combinations thereof with a compound including at least two hydroxy moieties with a (meth)acrylate moiety, wherein the esterification is catalyzed by an enzymatic catalyst
(b) capping at least one of the end of a polyester polyol with a (meth)acrylate moiety, wherein the end capping reaction is catalyzed by an enzymatic catalyst, and
(c) the combination of steps (a) and (b).

9. The method according to claim 8, wherein the enzyme catalyst comprises a lipase.

10. The method according to claim 8, wherein the enzyme catalyst for the capping step comprises a lipase.

11. The method according to any of claims 8 to 10, wherein the compound including at least two hydroxy moieties further comprises a double bond-containing moiety.

12. A surface covering coated with the composition according to any of the claims 1 to 7.

13. The surface covering according to claim 12, wherein the surface covering is a floor covering.

## Patentansprüche

1. Beschichtungszusammensetzung, umfassend ein (Meth)acrylatpolyesterpolyol, hergestellt nach einem Verfahren, das eine Stufe umfasst, ausgewählt aus der Gruppe, bestehend aus:
a) Bildung eines Polyesterpolyols durch Veresterung von polyfunktionellen Carbonsäuren und polyfunktionelle Hydroxy enthaltenden Stoffen mit einem (Meth)acrylat-Fragment, wobei die Veresterung durch enzymatische Katalyse katalysiert wird,
b) Absättigung wenigstens eines Endes eines Polyesterpolyols mit einem (Meth)acrylat-Fragment, wobei die Endabsättigungsreaktion durch einen enzymatischen Katalysator katalysiert wird,
c) die Kombination der Stufen a) und b).

2. Beschichtungszusammensetzung nach Anspruch 1, wobei die Zusammensetzung im Wesentlichen frei ist von Säurekatalysator- und Metallkatalysatorrückständen.

3. Beschichtungszusammensetzung nach Anspruch 1 oder 2, wobei der Enzymkatalysator ein Lipaseenzym umfasst.

4. Beschichtungszusammensetzung, umfassend eine Verbindung, ausgewählt aus der Gruppe, bestehend aus einem Urethanmonomer, wobei das Urethanfragment Urethanbindungen umfasst, gebildet aus wenigstens einem Hydroxyfragment an einem Polyesterpolyol nach einem der Ansprüche 1 bis 3, wobei das Polyesterpolyol durch enzymkatalysierte Veresterung hergestellt wird, mit wenigstens einem Isocyanatfragment an einem Polyisocyanat.

5. Beschichtungszusammensetzung nach Anspruch 4, wobei die Zusammensetzung ferner noch ein reaktionsfähiges Verdünnungsmittel umfasst.

6. Beschichtungszusammensetzung nach den Ansprüchen 4 oder 5, wobei die Zusammensetzung ferner noch ein Mattierungsmittel enthält.

7. Beschichtungszusammensetzung nach einem der Ansprüche 4 bis 6, wobei die Zusammensetzung ferner noch einen Photoinitiator enthält.

8. Verfahren zur Herstellung der Beschichtungszusammensetzung nach einem der Ansprüche 1 bis 3, umfassend
a) die Bildung eines Polyesterpolyols durch Veresterung einer Säure, ausgewählt aus der Gruppe, bestehend aus zweibasigen Carbonsäuren, mehrbasigen Carbonsäuren und Gemischen davon mit einer Verbindung, die wenigstens zwei Hydroxyfragmente mit einem (Meth)acrylat-Fragment umfasst, wobei die Veresterung durch einen enzymatischen Katalysator katalysiert wird,
b) Absättigung wenigstens eines Endes eines Polyesterpolyols mit einem (Meth)acrylat-Fragment, wobei die Endabsättigungsreaktion durch einen enzymatischen Katalysator katalysiert wird,
c) die Kombination der Stufen a) und b).

9. Verfahren nach Anspruch 8, wobei der Enzymkatalysator eine Lipase umfasst.

10. Verfahren nach Anspruch 8, wobei der Enzymkatalysator für die Absättigungsstufe eine Lipase umfasst.

11. Verfahren nach einem der Ansprüche 8 bis 10, wobei die wenigstens zwei HydroxyFragmente umfassende Verbindung außerdem noch ein eine Doppelbindung enthaltendes Fragment umfasst.

12. Oberflächenbelag, beschichtet mit der Zusammensetzung nach einem der Ansprüche 1 bis 7.

13. Oberflächenbelag nach Anspruch 12, wobei dieser ein Bodenbelag ist.

## Revendications

1. Composition de revêtement comprenant un polyester polyol (méth)acrylique, dans laquelle le polyester polyol est préparé par un procédé comprenant une étape choisie parmi le groupe consistant en :
(a) la formation d'un polyester polyol par estérification d'acides carboxyliques polyfonctionnels et de matières polyfonctionnelles contenant un hydroxy avec un groupe caractéristique (méth)acrylate, et dans laquelle l'estérification est catalysée par un catalyseur enzymatique,
(b) le coiffage d'au moins une de l'extrémité d'un polyester polyol avec un groupe caractéristique (méth)acrylate, dans laquelle la réaction de coiffage d'extrémité est catalysée par un catalyseur enzymatique, et
(c) la combinaison des étapes (a) et (b).

2. Composition de revêtement selon la revendication 1, dans laquelle la composition est essentiellement exempte de résidus de catalyseur acide et de résidus de catalyseur métallique.

3. Composition de revêtement selon la revendication 1 ou 2, dans laquelle le catalyseur enzymatique comprend une enzyme lipase.

4. Composition de revêtement comprenant un composé choisi parmi le groupe consistant en un monomère uréthane, dans laquelle le groupe caractéristique uréthane comprend des liaisons uréthane formées à partir d'au moins un groupe caractéristique hydroxy sur un polyester polyol selon l'une quelconque des revendications 1 à 3, dans laquelle le polyester polyol étant préparé par l'intermédiaire d'une estérification catalysée par une enzyme, avec au moins un groupe caractéristique isocyanate sur un polyisocyanate.

5. Composition de revêtement selon la revendication 4, dans laquelle la composition comprend en outre un diluant réactif.

6. Composition de revêtement selon les revendications 4 ou 5, dans laquelle la composition comprend en outre un agent de matité.

7. Composition de revêtement selon l'une quelconque des revendications 4 à 6, dans laquelle la composition comprend en outre un photoinitiateur.

8. Procédé de préparation de la composition de revêtement selon l'une quelconque des revendications 1 à 3, comprenant
(a) la formation d'un polyester polyol par estérification d'un acide choisi parmi le groupe consistant en un acide carboxylique dibasique, un acide carboxylique polybasique, et des combinaisons de ceux-ci avec un composé incluant au moins deux groupes caractéristiques hydroxy avec un groupe caractéristique (méth)acrylate, dans lequel l'estérification est catalysée par un catalyseur enzymatique,
(b) le coiffage d'au moins une de l'extrémité d'un polyester polyol avec un groupe caractéristique (méth)acrylate, dans lequel la réaction de coiffage d'extrémité est catalysée par un catalyseur enzymatique, et
(c) la combinaison des étapes (a) et (b).

9. Procédé selon la revendication 8, dans lequel le catalyseur enzymatique comprend une lipase.

10. Procédé selon la revendication 8, dans lequel le catalyseur enzymatique pour l'étape de coiffage comprend une lipase.

11. Procédé selon l'une quelconque des revendications 8 à 10, dans lequel le composé incluant au moins deux groupes caractéristiques hydroxy comprend en outre un groupe caractéristique contenant une liaison double.

12. Revêtement de surface revêtu avec la composition selon l'une quelconque des revendications 1 à 7.

13. Revêtement de surface selon la revendication 12, dans lequel le revêtement de surface est un revêtement de sol.
